# EUROPÄISCHE PATENTSCHRIFT

(11) **EP 3 868 418 B1**
(45) Veröffentlichungstag und Bekanntmachung des Hinweises auf die Patenterteilung: **28.06.2023**
(21) Anmeldenummer: 21158510.4
(22) Anmeldetag: 22.02.2021
(51) Int. Cl.: A61M 1/36

(54) **VORRICHTUNG UND VERFAHREN ZUM BILDEN EINES FLUIDKREISLAUFSYSTEMS SOWIE FLUIDKREISLAUFSYSTEM MIT EINER DERARTIGEN VORRICHTUNG**
DEVICE AND METHOD FOR FORMING A FLUID CIRCULATION SYSTEM AND FLUID CIRCULATION SYSTEM COMPRISING SUCH A DEVICE
DISPOSITIF ET PROCÉDÉ DE FORMATION D'UN SYSTÈME DE CIRCUIT DE FLUIDE, AINSI QUE SYSTÈME DE CIRCUIT DE FLUIDE DOTÉ D'UN TEL DISPOSITIF

(30) Priorität: 24.02.2020 DE 102020202351
(43) Veröffentlichungstag der Anmeldung: 25.08.2021
(73) Patentinhaber: Weber, Waldfried, 90556 Seukendorf (DE)
(72) Erfinder: Weber, Waldfried, 90556 Seukendorf (DE)
(74) Vertreter: Rau, Schneck & Hübner Patentanwälte Rechtsanwälte PartGmbB

(56) Entgegenhaltungen:
- DE-T2- 69 732 249
- GB-A- 2 439 144
- US-A1- 2018 154 066

## Beschreibung

Die vorliegende Patentanmeldung nimmt die Priorität der deutschen Patentanmeldung DE 10 2020 202 351.4 in Anspruch.

Die Erfindung betrifft eine Vorrichtung und ein Verfahren zum Bilden eines, insbesondere sterilen und geschlossenen, Fluidkreislaufsystems sowie ein Fluidkreislaufsystem mit einer derartigen Vorrichtung.

Bei der therapeutischen Apherese, die auch als Blutwäsche oder Blutreinigungsverfahren bezeichnet wird, werden Blut, Blutplasma oder Blutbestandteile eines Patienten in einem extrakorporalen Kreislauf, also außerhalb des Körpers des Patienten, therapeutisch behandelt, um pathogene oder überzählige Bestandteile wie Proteine, proteingebundene Substanzen und Zellen zu entfernen. Nach der Entfernung der Substanzen oder nach der Behandlung der Blutbestandteile wird das behandelte Blut, Blutplasma oder Blutbestandteile wieder zurückgeführt. Für die Bildung des extrakorporalen Kreislaufs sind verschiedene Komponenten erforderlich, die vor Ort in der Therapieeinrichtung manuell miteinander verbunden werden müssen. Bei unsachgemäßer Verbindung der Komponenten können Beschädigungen und/oder Verunreinigungen auftreten. Es besteht das Risiko, dass der extrakorporale Kreislauf nicht steril ausgeführt ist. Ein nicht steriler Kreislauf kann für die therapeutische Apherese nicht eingesetzt werden. Sofern die nicht sterile Ausführung nicht erkannt wird, stellt dieser extrakorporale Kreislauf ein erhöhtes Gesundheitsrisiko dar. Photophorese-Geräte sind aus GB 2 439 144 A und EP 3 693 036 A1 bekannt.

DE 697 32 249 T2 offenbart eine Kassette zum Steuern und Pumpen von Fluiden.

Der Erfindung liegt die Aufgabe zugrunde, das Gesundheitsrisiko bei der Bildung eines sterilen Fluidkreislaufsystems zu reduzieren und insbesondere den Aufwand beim Bilden eines, insbesondere sterilen und geschlossenen, Fluidkreislaufsystems zu reduzieren.

Diese Aufgabe wird durch die Merkmale der Patentansprüche 1, 10 und 11 gelöst. Erfindungsgemäß wurde erkannt, dass ein, insbesondere steriles und geschlossenes, Fluidkreislaufsystem mit einer Vorrichtung gebildet werden kann, bei der sämtliche Komponenten unlösbar miteinander verbunden sind. Die Vorrichtung ist einstückig ausgeführt. Die Vorrichtung ist in sich geschlossen und steril. Die Komponenten der Vorrichtung sind zerstörungsfrei nicht voneinander trennbar. Die Komponenten der Vorrichtung sind abgedichtet miteinander verbunden. Die Vorrichtung selbst ist in sich steril. Die Vorrichtung kann steril an ein Therapiegerät, insbesondere ein Apheresegerät-Blutschlauchsystem, das auch als Sammelkit bezeichnet wird, angeschlossen werden, wobei die Vorrichtung mit dem Therapiegerät einen, insbesondere zusätzlichen, insbesondere geschlossenen sterilen extrakorporalen Kreislauf bildet. Die Vorrichtung ist mit dem Therapiegerät steril koppelbar. Die Vorrichtung bildet einen Bestandteil eines Kreislaufsystems, wobei insbesondere eine Zuführleitung der Vorrichtung und eine Abführleitung der Vorrichtung mit dem Therapiegerät derart koppelbar sind, dass ein in sich geschlossenes Kreislaufsystem resultiert.

Insbesondere wird durch die sterile Verbindung der Vorrichtung mit dem Therapiegerät ein Fluidkreislaufsystem geschaffen, das eine sterile geschlossene Einheit bildet und geeignet ist, insbesondere Photophorese durchzuführen. Erfindungsgemäß wurde also gefunden, dass ein Therapiegerät, insbesondere ein Apheresegerät, durch die Vorrichtung mit einer sterilen geschlossenen Einheit erweitert werden kann. Mit der erfindungsgemäßen Vorrichtung kann ein bestehendes Apheresegerät unkompliziert, kosteneffizient und zuverlässig mit einem sterilen Fluidkreislaufsystem aufgewertet werden. Die erfindungsgemäße Vorrichtung ermöglicht eine unkomplizierte Nachrüstung und dadurch funktionelle Aufwertung des Apheresegeräts. Dadurch wird ein bestehendes Apheresegerät zu einem Photopheresegerät. Die Vorrichtung kann alternativ an eine Blutsammeleinrichtung oder einen Blutbeutel angeschlossen werden.

Die Vorrichtung umfasst eine Zuführleitung, mittels der ein Fluid der Vorrichtung zugeführt werden kann. Die Zuführleitung ist insbesondere als Schlauchleitung ausgeführt. Die Schlauchleitung ist insbesondere aus einem Kunststoffmaterial hergestellt, das insbesondere biokompatibel ist und insbesondere bezüglich Material und Durchmesser dem Schlauch des Apheresegerät-Blutschlauchsystems angepasst ist.

Die Schlauchleitung ist insbesondere aus Polyvinylchlorid (PVC) hergestellt. Die weiteren Komponenten der Vorrichtung sind insbesondere aus einem Kunststoffmaterial hergestellt, insbesondere aus Acryl-Material, Polypropylen (PP), Polyamid (PA), Ethylen-Vinylacetat-Copolymer (EVA), insbesondere Polyethylen-Vinyl-Acetat, und/oder Acrylnitril-Butadien-Styrol-Copolymer (ABS).

Insbesondere sind alle für die Vorrichtung verwendeten Materialien Kunststoffmaterialien, die insbesondere weichmacherfrei sind, insbesondere frei von Phthalat und insbesondere frei von Diethylhexylphthalat (DEHP).

Die Zuführleitung weist insbesondere ein integriertes Filterelement, insbesondere in Form eines Koagelfilters, auf.

Die Zuführleitung umfasst ein erstes Anschlusselement. Mit dem ersten Anschlusselement ist die Vorrichtung an das Therapiegerät steril anschließbar. Das erste Anschlusselement kann durch ein steriles Ende der Zuführleitung ausgeführt sein, wobei die Zuführleitung mit einer korrespondierenden Leitung des Therapiegeräts mittels eines sterilen Andocksystems, insbesondere eines Leitungsschweißgeräts durch Schweißen, verbunden werden kann. Das sterile Ende der Zuführleitung ist dadurch gebildet, dass die Zuführleitung an dem ersten Anschlusselement geschlossen ist, wobei der Verschluss an diesem Ende steril ausgeführt ist. Alternativ kann das erste Anschlusselement als Dorn zum Einstechen in ein korrespondierendes Anschlusselement des Therapiegeräts ausgeführt sein. Das erste Anschlusselement kann auch als Luer-Lock-Verbindung ausgeführt sein.

Die Vorrichtung weist einen Sammelbehälter auf, der an die Zuführleitung angeschlossen ist. Der Sammelbehälter ist insbesondere als Sammelbeutel aus einem Kunststoffmaterial hergestellt. Das Kunststoffmaterial ist biokompatibel. Der Sammelbeutel ist insbesondere aus Ethylen-Vinylacetat-Copolymer (EVA), insbesondere aus Polyethylen-Vinyl-Acetat, hergestellt.

Der Sammelbeutel weist insbesondere ein Volumen von 100 ml bis 1000 ml auf.

An den Sammelbehälter ist mittels einer Verbindungsleitung ein Behandlungsbehälter angeschlossen. Der Behandlungsbehälter ist insbesondere als Behandlungsbeutel aus einem Kunststoffmaterial hergestellt. Der Behandlungsbeutel ist insbesondere aus demselben Kunststoffmaterial hergestellt, aus dem der Sammelbehälter hergestellt ist. Der Behandlungsbeutel ermöglicht eine Behandlung des Fluids. Der Behandlungsbeutel ist insbesondere als Bestrahlungsbeutel ausgeführt. Der Bestrahlungsbeutel besteht insbesondere aus einem Kunststoffmaterial, das in einen Wellenlängenbereich von 100 nm bis 1000 nm, insbesondere von 150 nm bis 900 nm transparent ist. Transparent bedeutet, dass der Transmissionsgrad T für das Material des Bestrahlungsbeutels für Licht in dem genannten Wellenlängenbereich mindestens 30 %, insbesondere mindestens 50 %, insbesondere mindestens 70 %, insbesondere mindestens 80 %, insbesondere mindestens 90 % und insbesondere mindestens 95 % beträgt.

Der Bestrahlungsbeutel ermöglicht die Bestrahlung des Fluids mit Licht unterschiedlicher Wellenlängen. Insbesondere wird das Fluid in dem Bestrahlungsbeutel mäanderförmig geführt. Insbesondere ist die Innenfläche des Fluidströmungskanal in dem Bestrahlungsbeutel mit einer Oberflächenstrukturierung ausgeführt, insbesondere geriffelt. Dadurch werden bei der Durchströmung des Bestrahlungsbeutels in dem Fluid Mikroturbulenzen erzeugt, sodass Teilchen in dem Fluid, insbesondere Zellen, in Rotation, insbesondere um eine körpereigene Achse, versetzt werden. Dadurch gelingt es, die Teilchen in dem Fluid aus verschiedenen Richtungen zu bestrahlen. Insbesondere ist es denkbar, dass die Teilchen eine vollständige, also 360°-Drehung um die eigene Achse durchführen. Das Bestrahlungsergebnis ist dadurch verbessert. Dadurch ist eine gezielte und definierte Behandlung einer erhöhten Menge des Fluids in dem Behandlungsbeutel möglich. Insbesondere kann Blut mit einem erhöhten Hämatokrit, also mit einem erhöhten Anteil von roten Blutkörperchen am Volumen des Blutes, vorteilhaft bestrahlt werden. Das Risiko einer Abschattung von Teilchen in dem Fluid, insbesondere aufgrund der erhöhten Anzahl der roten Blutkörperchen, ist reduziert und insbesondere vermieden. Ein derartiger Bestrahlungsbeutel ermöglicht die Behandlung von Blut mit unterschiedlichen Hämatokriten, insbesondere in einem Bereich von 0 % bis 10 % und insbesondere von 1 % bis 6 %. Derartige Hämatokriten sind typisch für vorbehandeltes Blut, in dem noch ein Restanteil an roten Blutkörperchen vorhanden ist. Der Bestrahlungsbeutel ist auch geeignet, Flüssigkeiten mit Verunreinigungen zu bestrahlen.

Die Vorrichtung weist ferner eine mit dem Behandlungsbehälter verbundene Abführleitung auf, die ein zweites Anschlusselement umfasst. Insbesondere weist die Abführleitung ein integriertes Filterelement in Form eines Koagelfilters auf. Mit dem zweiten Anschlusselement ist die Vorrichtung mit einem korrespondierenden Element des Therapiegeräts und/oder mit dem Patienten steril verbindbar. Das zweite Anschlusselement kann entsprechend dem ersten Anschlusselement ausgeführt sein. Das zweite Anschlusselement kann auch als Luer-Anschluss ausgeführt sein. Die Abführleitung dient zum Rückführen des durch die Vorrichtung geförderten Fluids in das Therapiegerät und/oder zu dem Patienten. Die Abführleitung kann analog der Zuführleitung als Schlauchleitung aus einem Kunststoffmaterial ausgeführt sein.

Die erfindungsgemäße Vorrichtung ist insbesondere ein einteiliges System, das mit dem ersten Anschlusselement und dem zweiten Anschlusselement an das Therapiegerät angeschlossen werden kann. Das Risiko einer unsachgemäßen Verbindung der Komponenten der Vorrichtung ist ausgeschlossen. Ein Gesundheitsrisiko ist ausgeschlossen. Die Vorrichtung und das Therapiegerät bilden ein geschlossenes, steriles Fluidkreislaufsystem.

Die Vorrichtung ist insbesondere ein in sich vollständiger und insbesondere steriler Bausatz, der insbesondere universell an das Therapiegerät angedockt werden kann. Die Vorrichtung ist insbesondere universell auf das Therapiegerät adaptierbar und kann insbesondere für bereits existierende extrakorporale Fluidkreisläufe und beispielsweise für in Beuteln gesammelte Körperflüssigkeiten wie Blut und Blutbestandteile verwendet werden. Mit der erfindungsgemäßen Vorrichtung und einem Apheresegerät kann ein Fluidkreislaufsystem in Form eines Apharese-Systems gebildet werden. Insbesondere kann das Apherese-System als Photopherese-System zur Photo-Immun-Therapie genutzt werden. Die Vorrichtung ist insbesondere als Schlauchsystem hergestellt.

Die Vorrichtung umfasst eine Pumpe, mittels der das Fluid in der Vorrichtung gefördert werden kann. Insbesondere ist die Pumpe entlang der Verbindungsleitung, also insbesondere zwischen dem Sammelbehälter und dem Behandlungsbehälter angeordnet.

Ein Probenentnahmebehälter, der an dem Sammelbehälter angeschlossen ist, ermöglicht eine definierte und fehlerunanfällige Probenentnahme des Fluids.

Die Fluidzufuhr von dem Sammelbehälter in den jeweiligen Probenentnahmebehälter ist jeweils, insbesondere unabhängig voneinander, mittels eines Sperrelements, insbesondere in Form einer Klemme, schließbar. Es ist möglich, unabhängige Proben des Fluids vor und nach einer Therapie zu entnehmen. Die Probenentnahmebehälter weisen insbesondere jeweils ein Volumen auf, das insbesondere mindestens 5 ml und insbesondere höchstens 10 ml beträgt. Die Probenentnahmebehälter sind insbesondere jeweils als Beutel ausgeführt.

Eine Rückführleitung gemäß Anspruch 2 ermöglicht die Rückführung des in dem Behandlungsbehälter behandelten Fluids in den Sammelbehälter. Das Fluid rezirkuliert in den Sammelbehälter. Der Sammelbehälter wird auch als Rezirkulationsbehälter oder als Rezirkulationsbeutel bezeichnet. Es ist möglich, unterschiedliche Volumina des Fluids in dem Behandlungsbehälter zur Behandlung bereitzustellen.

Eine Sekundärzuführleitung gemäß Anspruch 3 ermöglicht die sterile Zuführung von Flüssigkeiten jeglicher Art in den Sammelbehälter. Insbesondere weist die Sekundärzuführleitung ein integriertes Filterelement auf, das eine Porengröße von höchstens 0,2 µm, insbesondere von höchstens 0,1 µm und insbesondere von höchstens 0,05 µm aufweist. Die Sekundärzuführleitung ist insbesondere unmittelbar an den Sammelbehälter angeschlossen. An einem dem Sammelbehälter abgewandten Ende kann die Sekundärzuführleitung ein Anschlusselement aufweisen, mit dem die Sekundärzuführleitung steril an eine Quelle der zuzuführenden Flüssigkeit anschließbar ist. Als Quelle kann beispielsweise ein Vorratsbehälter der Flüssigkeit dienen.

Eine Spülleitung gemäß Anspruch 4 ermöglicht ein unmittelbares, unkompliziertes und zuverlässiges Spülen, das auch als Primen bezeichnet wird, der Vorrichtung, insbesondere mit physiologischen Spüllösungen. Die Spülleitung ist mittels eines dritten Anschlusselements, das insbesondere analog zu dem ersten Anschlusselement ausgeführt ist, mit einer Quelle der Spüllösung steril verbindbar. In der Spülleitung ist insbesondere ein Filterelement, insbesondere ein Bakterienfilter, insbesondere mit einer Porengröße von höchstens 0,2 µm, insbesondere von höchstens µm und insbesondere von höchstens 0,05 µm, integriert. Die Spülleitung ist an die Verbindungsleitung, insbesondere mittels eines Y-Verbindungselements angeschlossen.

Ein Verschlusselement gemäß Anspruch 5 vereinfacht die Festlegung einer Spülrichtung in der Vorrichtung. Insbesondere ist das Verschlusselement entlang der Fluidströmungsrichtung stromaufwärts zu dem Y-Verbindungselement an der Verbindungsleitung angeordnet. Die Strömungsrichtung der Spüllösung ist der Fluidströmungsrichtung gleichgerichtet.

Ein Steuerelement gemäß Anspruch 6 dient zum Steuern und insbesondere zum Regeln der Durchflussrate des Fluids in die Vorrichtung, innerhalb der Vorrichtung und/oder aus der Vorrichtung heraus.

Eine Vorrichtung gemäß Anspruch 7 ermöglicht die Rückführung des Fluids zu dem Therapiegerät aus dem Sammelbehälter.

Gemäß Anspruch 8 sind mindestens zwei Probenentnahmebehälter vorgesehen, die an den Sammelbehälter angeschlossen sind. Die Probenentnahmebehälter sind insbesondere identisch ausgeführt und weisen jeweils ein Volumen auf, das kleiner ist als das des Sammelbehälters.

Eine Vorrichtung gemäß Anspruch 9 reduziert das Gesundheitsrisiko zusätzlich.

Ein Fluidkreislaufsystem gemäß Anspruch 10 ist zuverlässig steril ausgeführt. Das Fluidkreislaufsystem mit der erfindungsgemäßen Vorrichtung ermöglicht verschiedene Anwendungen. Als Therapiegerät dient insbesondere ein Apheresegerät. Insbesondere kann das Fluidkreislaufsystem durch eine Bestrahlungseinheit zur Bestrahlung des Bestrahlungsbeutels ergänzt werden. Ein derartiges Fluidkreislaufsystem kann für die Durchführung der Photopherese verwendet werden.

Ein Verfahren gemäß Anspruch 11 ermöglicht die unkomplizierte und zuverlässige Bildung eines Fluidkreislaufsystems.

Sowohl die in den Patentansprüchen angegebenen Merkmale als auch die in dem nachfolgenden Ausführungsbeispiel der erfindungsgemäßen Vorrichtung angegebenen Merkmale sind jeweils für sich alleine oder in Kombination miteinander geeignet, den erfindungsgemäßen Gegenstand weiterzubilden. Die jeweiligen Merkmalskombinationen stellen hinsichtlich der Weiterbildungen des Erfindungsgegenstandes keine Einschränkung dar, sondern weisen im Wesentlichen lediglich beispielhafte Charakter auf.

Weitere Merkmale, Vorteile und Einzelheiten der Erfindung ergeben sich aus der nachfolgenden Beschreibung eines Ausführungsbeispiels anhand der Zeichnung. Es zeigen:

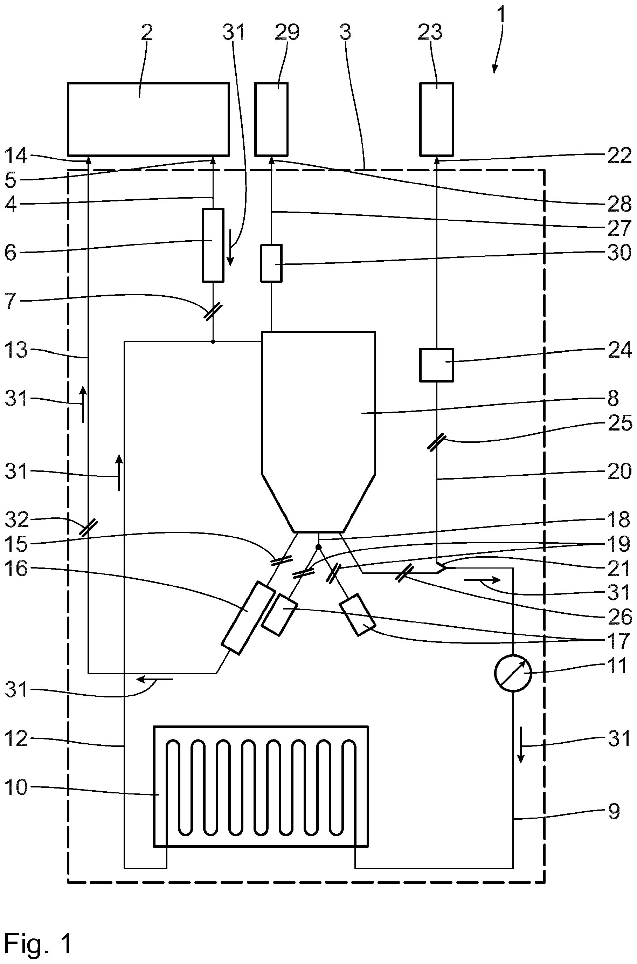

- Fig. 1: eine schematische Darstellung eines Fluidkreislaufsystems mit einem Therapiegerät und einer erfindungsgemäßen Vorrichtung.

Ein in Fig. 1 als Ganzes mit 1 gekennzeichnetes Fluidkreislaufsystem umfasst ein Therapiegerät 2, das gemäß dem gezeigten Ausführungsbeispiel als Apheresegerät ausgeführt ist. Das Fluidkreislaufsystem 1 umfasst ferner eine an das Therapiegerät 2 angeschlossene Vorrichtung 3, wie in Fig. 1 schematisch durch den gestrichelten Rahmen symbolisiert ist.

Die Vorrichtung 3 umfasst eine als Kunststoffschlauch ausgeführte Zuführleitung 4, die mittels eines ersten Anschlusselements 5, das gemäß dem gezeigten Ausführungsbeispiel als Einstechdorn ausgeführt ist, zuverlässig und steril mit einem korrespondierenden, nicht dargestellten Anschlusselement des Therapiegeräts 2 verbunden ist. An der Zuführleitung 4 ist ein erstes Filterelement 6 in Form eines Koagelfilters angeordnet. Das erste Filterelement 6 ist optional, kann also auch entfallen.

Entlang der Zuführleitung 4 ist ein erstes Steuerelement 7 angeordnet. Das erste Steuerelement 7 ist gemäß dem gezeigten Ausführungsbeispiel als veränderlich einstellbare Klemme ausgeführt. Mit dem ersten Steuerelement kann der Innenquerschnitt der Zuführleitung 4 und damit die Durchflussrate eines Fluids durch die Zuführleitung 4 veränderlich eingestellt werden.

Die Zuführleitung 4 ist an einen Sammelbehälter 8 angeschlossen. Der Sammelbehälter 8 ist als Kunststoffbeutel ausgeführt. Der Sammelbehälter 8 wird auch als Sammelbeutel bezeichnet.

Der Sammelbeutel 8 ist mittels einer Verbindungsleitung 9 mit einem Behandlungsbehälter 10 verbunden. Entlang der Verbindungsleitung 9 ist eine Fluidpumpe 11 angeordnet, um das Fluid in dem Fluidkreislaufsystem 1, insbesondere innerhalb der Vorrichtung 3, zu fördern. Die Verbindungsleitung 9 ist als Schlauchleitung aus einem Kunststoffmaterial hergestellt.

Der Behandlungsbehälter 10 ist als Kunststoffbeutel ausgeführt. Gemäß dem gezeigten Ausführungsbeispiel ist der Behandlungsbehälter 10 derart ausgeführt, dass das darin, insbesondere mäanderförmig, geführte Fluid mittels einer nicht dargestellten Bestrahlungseinheit mit Licht in einem Wellenlängenbereich von UV-Licht bis IR-Licht bestrahlt werden kann. Dazu ist der Behandlungsbehälter 10 aus einem in dem genannten Wellenlängenbereich transparenten Material ausgeführt. Der Behandlungsbehälter 10 wird auch als Bestrahlungsbeutel bezeichnet.

Der Bestrahlungsbeutel 10 ist über eine Rückführleitung 12 an den Sammelbeutel 8 angeschlossen. Gemäß dem gezeigten Ausführungsbeispiel mündet die Zuführleitung 4 in die Rückführleitung 12. Es ist aber auch denkbar, dass die Zuführleitung 4 und die Rückführleitung 12 jeweils separat in den Sammelbeutel 8 geführt sind. Insbesondere sind die Zuführleitung 4 und/oder die Rückführleitung 12 in einem oberen Bereich des Sammelbeutels 8 angeordnet, sodass das Fluid infolge der Schwerkraft selbsttätig in dem Sammelbeutel 8 nach unten gelangen und über die in den unteren Bereich des Sammelbeutels 8 angeschlossene Verbindungsleitung 9 abgeführt werden kann.

Ebenfalls im unteren Bereich des Sammelbeutels 8 ist eine Abführleitung 13 an dem Sammelbeutel 8 angeschlossen. Die Abführleitung 13 ist über den Sammelbeutel 8 und die Rückführleitung 12 mit dem Bestrahlungsbeutel 10 verbunden. Die Abführleitung 13 weist ein zweites Anschlusselement 14 auf, das gemäß dem gezeigten Ausführungsbeispiel als Luer-Anschluss ausgeführt ist. Mit dem zweiten Anschlusselement 14 ist die Vorrichtung 3 an einen korrespondierenden Luer-Anschluss des Therapiegeräts 2 zuverlässig und steril anschließbar. Das zweite Anschlusselement 14 kann alternativ auch über eine Kanüle an den Patienten direkt angeschlossen werden.

Entlang der Abführleitung 13 ist ein zweites Steuerelement 15 vorgesehen, das insbesondere identisch zu dem ersten Steuerelement 7 ausgeführt ist.

Entlang der Abführleitung 13 ist ein zweites Filterelement 16, insbesondere integriert, angeordnet. Das zweite Filterelement 16 ist gemäß dem gezeigten Ausführungsbeispiel als Koagelfilter ausgeführt.

Entlang der Abführleitung 13 ist ein viertes Steuerelement 32 vorgesehen, das insbesondere identisch zu dem ersten Steuerelement 7 ausgeführt ist.

Das vierte Steuerelement 32 ist insbesondere entlang der Fluidströmungsrichtung 31 zwischen dem zweiten Filterelement 16 und dem zweiten Anschlusselement 14 angeordnet. Das vierte Steuerelement 32 ist insbesondere als veränderlich einstellbare Klemme ausgeführt. Das vierte Steuerelement 32 dient dazu, den Rückfluss aus der Vorrichtung 3 infolge der Gravitation zu dem Therapiegerät 2 und/oder direkt zum Patienten, insbesondere den Volumenstrom des Fluids, zu steuern. Das vierte Steuerelement 32 kann auch als Pumpe, insbesondere als geregelte Pumpe, ausgeführt sein.

An dem Sammelbeutel 8 sind zwei, insbesondere identisch ausgeführte, Probenentnahmebehälter 17 über eine Probenentnahmeleitung 18 angeschlossen. Die Probenentnahmeleitung 18 ist als Schlauchleitung aus einem Kunststoffmaterial ausgeführt. Die Probenentnahmebehälter 17 sind jeweils als Kunststoffbeutel ausgeführt, die ein Volumen von mindestens 5 ml und höchstens 10 ml aufweisen. Entlang der Probenentnahmeleitung 18, die sich auf die einzelnen Probenentnahmebehälter 17 verzweigt, ist jeweils ein Sperrelement 19 in Form einer Klemme angeordnet, um den Fluidfluss aus dem Sammelbehälter 8 in den jeweiligen Probenentnahmebehälter 17 zu sperren bzw. freizugeben.

Die Vorrichtung 3 weist ferner eine Spülleitung 20 auf, die als Schlauchleitung aus einem Kunststoffmaterial ausgeführt ist. Die Spülleitung 20 ist mittels eines Y-Verbindungselements 21 an die Verbindungsleitung 9 angeschlossen. Die Spülleitung 20 dient zum Spülen der Vorrichtung 3 mit einer Spüllösung. Dazu ist die Spülleitung 20 mittels eines dritten Anschlusselements 22 an eine Spüllösungsquelle 23 angeschlossen. Die Spüllösungsquelle 23 ist insbesondere ein Vorratsbehälter, in dem Spüllösung bevorratet ist. Das dritte Anschlusselement 22 ist insbesondere in Form eines einstechbaren Dorns ausgeführt, der in die Spüllösungsquelle 23 unmittelbar eingesteckt wird. Mittels des Anschlusselements ist die Spülleitung 20 an die Spüllösungsquelle 23 steril angeschlossen.

Entlang der Spülleitung 20 ist ein drittes Filterelement 24 in Form eines Bakterienfilters angeordnet. Das dritte Filterelement 24 ist insbesondere ein Sterilfilter. Insbesondere ist der Bakterienfilter in der Spülleitung 20 integriert ausgeführt. Entlang der Spülleitung 20 ist ein drittes Steuerelement 25 angeordnet, das insbesondere identisch zu dem ersten Steuerelement 7 und/oder dem zweiten Steuerelement 15 ausgeführt ist. Das dritte Steuerelement 25 ist insbesondere an der Spülleitung 20 zwischen dem dritten Filterelement 24 und dem Y-Verbindungselement 21 angeordnet.

An der Verbindungsleitung 9 ist ein Verschlusselement 26 in Form einer Klemme angeordnet. Das Verschlusselement 26 ist bezogen auf die Fluidströmungsrichtung 31 in der Verbindungsleitung 9 stromaufwärts zu dem Y-Verbindungselement 21 angeordnet.

An den Sammelbeutel 8 ist eine Sekundärzuführleitung 27 angeschlossen, die mittels eines vierten Anschlusselements 28 mit einer Flüssigkeitsquelle 29 steril verbunden ist. Entlang der Sekundärzuführleitung 27 ist ein, insbesondere integriert ausgeführtes, viertes Filterelement 30 angeordnet. Das vierte Filterelement 30 ist insbesondere als Sterilfilter ausgeführt.

Insbesondere sind sämtliche Leitungen der Vorrichtung 3, insbesondere die Zuführleitung 4, die Verbindungsleitung 9, die Rückführleitung 12, die Abführleitung 13, die Probenentnahmeleitung 18, die Spülleitung 20 und/oder die Sekundärzuführleitung 27, jeweils als Schlauchleitung aus einem Kunststoffmaterial ausgeführt. Das verwendete Kunststoffmaterial ist insbesondere biokompatibel.

Insbesondere sind die in der Vorrichtung 3 verwendeten Behälter, insbesondere der Sammelbehälter 8, der Bestrahlungsbeutel 10 und/oder die Probenentnahmebehälter 17, als Kunststoffbeutel ausgeführt.

Bei der Vorrichtung 3 sind sämtliche Komponenten fest und unlösbar miteinander verbunden. Ein unbeabsichtigtes Lösen von Komponenten der Vorrichtung 3 ist ausgeschlossen. Die Vorrichtung 3 ist in sich steril ausgeführt. Die Vorrichtung 3 ist abgedichtet und über die Anschlusselemente 5, 14, 22 und 28 mit externen Komponenten 2, 23, 29 steril verbindbar.

Insbesondere sind das erste Steuerelement 7, das zweite Steuerelement 15, das dritte Steuerelement 25 und das Verschlusselement 26 als reine Sperrelemente ausgeführt und insbesondere identisch zu dem Sperrelement 19 ausgeführt. Die Sperrelemente 7, 15, 19, 25 und 26 sind insbesondere als sperrende Klemmen ausgeführt.

Nachfolgend wird die Funktion des Fluidkreislaufsystems 1 näher erläutert.

Von dem Therapiegerät 2 wird Blut eines Patienten zur Behandlung in der Vorrichtung 3 bereitgestellt. Das Blut wird von dem Therapiegerät 2 über die Zuführleitung 4 dem Sammelbeutel 8 zugeführt. Aus dem Sammelbeutel 8 gelangt das Blut über die Verbindungsleitung 9 in den Bestrahlungsbeutel 10.

In dem Bestrahlungsbeutel 10 findet eine Behandlung des Blutes, beispielsweise eine Bestrahlung mit Licht, statt. Das behandelte Blut wird aus dem Bestrahlungsbeutel 10 über die Rückführleitung 12 in den Sammelbeutel 8 rückgeführt und gegebenenfalls für eine weitere Behandlung über die Verbindungsleitung 9 in den Bestrahlungsbeutel 10 zirkuliert. Die Förderung des Blutes innerhalb der Vorrichtung 3 erfolgt mittels der Fluidpumpe 11.

Nach Abschluss der Behandlung des Blutes wird dieses aus dem Sammelbeutel 8 über die Abführleitung 13 an das Therapiegerät 2 zurückgeführt. Zu diesem Zweck kann das Steuergerät 15 geöffnet werden. Solange keine Rückführung des Blutes in das Therapiegerät 2 erfolgt, ist die Abführleitung 13 mittels des zweiten Steuerelements 15 abgeschlossen.

Im regulären Betrieb der Vorrichtung 3, also während Blut durch die Vorrichtung 3, insbesondere durch die Verbindungsleitung 9, fließt, ist das Verschlusselement 26 geöffnet, erlaubt also einen Durchfluss von Blut durch die Verbindungsleitung 9.

Das Verschlusselement 26 wird geschlossen, wenn eine Spülung der Vorrichtung 3 erfolgt. Zu diesem Zweck wird eine Spüllösung aus der Spüllösungsquelle 23 über die Spülleitung 20 der Verbindungsleitung 9 zugeführt. Die Spüllösung kann durch sämtliche Komponenten der Vorrichtung 3 fließen.

Die Probenentnahmebehälter 17, die unabhängig voneinander ausgeführt sind, ermöglichen eine Probenentnahme insbesondere vor und nach einer Behandlung.

Über die Sekundärzuführleitung kann eine Flüssigkeit aus der Flüssigkeitsquelle 29 dem Sammelbehälter 8 zugeführt werden. Als Flüssigkeit dient insbesondere ein Photoaktuator, wie beispielsweise Psoralene, Verteporfin, Vitamin B 12, der sich an die DNA der gesammelten Blutzellen anlagert.

Zusätzlich oder alternativ handelt es sich um eine Flüssigkeit, wie beispielsweise eine Aminosäure und/oder Enzyme, die nach einer Inkubation mit den Blutzellen einen natürlichen Photoaktuator produzieren, der sich entsprechend an die DNA der gesammelten Blutzellen anlagert.

Nachfolgend wird ein Verfahren zum Bilden des Fluidkreislaufsystems mit der Vorrichtung 3 und dem Apheresegerät 2 näher erläutert.

Zunächst wird die Vorrichtung 3 bereitgestellt. Die Vorrichtung 3 ist ein steriles System. Die Vorrichtung 3 selbst ist kein Kreislaufsystem und ist insofern "offen" ausgeführt. Offen bedeutet, dass die Vorrichtung 3 die Zuführleitung 4 und die Abführleitung 13 aufweist. Dadurch dass diese Leitungen 4, 14 mit den jeweils daran angeordneten Anschlusselementen 5, 14 mit dem Apheresegerät 2 koppelbar sind, kann ein geschlossenes Kreislaufsystem gebildet werden. Die Bildung eines geschlossenen, sterilen Kreislaufsystems ist unkompliziert und insbesondere unabhängig von dem jeweiligen Therapiegerät möglich. Die Vorrichtung 3 garantiert eine unkomplizierte, zuverlässige flexible Möglichkeit zur Schaffung eines sterilen Fluidkreislaufs. Darüber hinaus wird das Apheresegerät 2 bereitgestellt. Die in sich geschlossene und sterile Vorrichtung 3 wird mit den Anschlusselementen 5, 14 jeweils steril an das Apheresegerät 2 angeschlossen. Die Anschlusselemente 5, 14 sind steril ausgeführt. Das Risiko einer Verunreinigung der Koppelstellen, an welchen die Anschlusselemente 5, 14 an das Apheresegerät 2 gekoppelt sind, ist ausgeschlossen. Es ist gewährleistet, dass das Fluidkreislaufsystem in sich geschlossen und in sich steril ist.

## Patentansprüche

1. Vorrichtung zum Bilden eines Fluidkreislaufsystems, wobei die Vorrichtung (3) umfasst
a. eine Zuführleitung (4) mit einem ersten Anschlusselement (5),
b. einen an die Zuführleitung (4) angeschlossenen Sammelbehälter (8),
c. eine Pumpe (11) zum Fördern des Fluides,
d. einen an den Sammelbehälter (8) mittels einer Verbindungsleitung (9) angeschlossenen Bestrahlungsbeutel (10),
e. eine mit dem Bestrahlungsbeutel (10) verbundene Abführleitung (13) mit einem zweiten Anschlusselement (14),
wobei sämtliche Komponenten der Vorrichtung (3) unlösbar miteinander verbunden sind,
**dadurch gekennzeichnet, dass** mindestens ein Probenentnahmebehälter (17) an dem Sammelbehälter (8) angeschlossen ist.

2. Vorrichtung gemäß Anspruch 1, **dadurch gekennzeichnet, dass** der Bestrahlungsbeutel (10) mittels einer Rückführleitung (12) an den Sammelbehälter (8) angeschlossen ist.

3. Vorrichtung gemäß einem der vorstehenden Ansprüche, **dadurch gekennzeichnet, dass** an den Sammelbehälter (8) eine Sekundärzuführleitung (27) angeschlossen ist, über einen Sterilfilter (30).

4. Vorrichtung gemäß einem der vorstehenden Ansprüche, **gekennzeichnet durch** eine an die Verbindungsleitung (9) angeschlossene Spülleitung (20), die ein drittes Anschlusselement (22) aufweist, das mittels eines Sterilfilters (24) mit der Spülleitung (20) verbunden ist.

5. Vorrichtung gemäß einem der vorstehenden Ansprüche, **dadurch gekennzeichnet, dass** entlang der Verbindungsleitung (9) ein Verschlusselement (26) zum Absperren der Verbindungsleitung (9) angeordnet ist.

6. Vorrichtung gemäß einem der vorstehenden Ansprüche, **dadurch gekennzeichnet, dass** an der Zuführleitung (4), an der Abführleitung (13) und/oder an der Spülleitung (20) jeweils ein Steuerelement (7, 15, 25, 42) zum Steuern der Durchflussrate des Fluides angeordnet ist.

7. Vorrichtung gemäß einem der vorstehenden Ansprüche, **dadurch gekennzeichnet, dass** die Abführleitung (13) unmittelbar an dem Sammelbehälter (8) angeschlossen ist, insbesondere mittels eines Koagelfilters (16).

8. Vorrichtung gemäß einem der vorstehenden Ansprüche, **dadurch gekennzeichnet, dass** mindestens zwei Probenentnahmebehälter (17) an dem Sammelbehälter (8) angeschlossen sind.

9. Vorrichtung gemäß einem der vorstehenden Ansprüche, **dadurch gekennzeichnet, dass** die Vorrichtung (3) steril verschlossen ist.

10. Fluidkreislaufsystem mit einem Therapiegerät (2) und mit einer daran steril angeschlossenen Vorrichtung (3) gemäß einem der vorstehenden Ansprüche.

11. Verfahren zum Bilden eines, insbesondere sterilen geschlossenen Fluidkreislaufsystems, **gekennzeichnet durch** die Verfahrensschritte
- Bereitstellen einer Vorrichtung gemäß einem der Ansprüche 1 bis 9, steriles Koppeln der Vorrichtung (3) mit einem Therapiegerät (2) insbesondere einem Apheresegerät, wobei das sterile Koppeln dadurch erfolgt, dass das erste Anschlusselement (5) und das zweite Anschlusselement (14) steril an das Therapiegerät (2) angeschlossen und dadurch ein geschlossenes, steriles Fluidkreislaufsystem gebildet werden.

## Claims

1. Apparatus for forming a fluid circulation system, the apparatus (3) comprising
a. a supply line (4) with a first connection element (5),
b. a collection container (8) that is connected to the supply line (4),
c. a pump (11) to convey the fluid,
d. an irradiation bag (10) that is connected to the collection container (8) by means of a connection line (9),
e. a discharge line (13) that is connected to the irradiation bag (10), with a second connection element (14),
wherein all components of the apparatus (3) are non-detachably connected to each other,
**characterized in that** at least one sampling container (17) is connected to the collection container (8).

2. Apparatus according to claim 1, **characterized in that** the irradiation bag (10) is connected to the collection container (8) by means of a return line (12).

3. Apparatus according to any one of the preceding claims, **characterized in that** a secondary supply line (27) is connected to the collection container (8), via a sterile filter (30).

4. Apparatus according to any one of the preceding claims, **characterized by** a flushing line (20) that is connected to the connection line (9) and has a third connection element (22) that is connected to the flushing line (20) by means of a sterile filter (24).

5. Apparatus according to any one of the preceding claims, **characterized in that** a closure element (26) to shut off the connection line (9) is arranged along the connection line (9).

6. Apparatus according to any one of the preceding claims, **characterized in that** a control element (7, 15, 25, 42) to control the flow rate of the fluid is arranged at the supply line (4), at the discharge line (13) and/or at the flushing line (20).

7. Apparatus according to any one of the preceding claims, **characterized in that** the discharge line (13) is connected directly to the collection container (8), in particular by means of a coagulum filter (16).

8. Apparatus according to any one of the preceding claims, **characterized in that** at least two sampling containers (17) are connected to the collecting container (8).

9. Apparatus according to any one of the preceding claims, **characterized in that** the apparatus (3) is sterilely sealed.

10. Fluid circulation system comprising a therapy device (2) and comprising an apparatus (3) that is sterilely connected thereto according to any one of the preceding claims.

11. Method for forming a closed fluid circulation system, in particular a sterile one, **characterized by** the following method steps
- providing an apparatus according to any one of claims 1 to 9, sterilely coupling the apparatus (3) to a therapy device (2), in particular an apheresis machine, wherein the sterile coupling is performed by sterilely connecting the first connection element (5) and the second connection element (14) to the therapy device (2), thereby forming a closed, sterile fluid circulation system.

## Revendications

1. Dispositif pour former un système de circulation de fluide, le dispositif (3) comprenant
a. une conduite d'alimentation (4) avec un premier élément de raccordement (5),
b. un récipient collecteur (8) raccordé à la conduite d'alimentation (4),
c. une pompe (11) pour refouler le fluide,
d. un sac d'irradiation (10) raccordé au récipient collecteur (8) au moyen d'une conduite de liaison (9),
e. une conduite d'évacuation (13) reliée au sac d'irradiation (10) et comportant un deuxième élément de raccordement (14),
tous les composants du dispositif (3) étant reliés entre eux de manière indissociable,
**caractérisé en ce qu'**au moins un récipient de prélèvement d'échantillons (17) est raccordé au récipient collecteur (8).

2. Dispositif selon la revendication 1, **caractérisé en ce que** le sac d'irradiation (10) est raccordé au récipient collecteur (8) au moyen d'une conduite de retour (12).

3. Dispositif selon l'une quelconque des revendications précédentes, **caractérisé en ce qu'**une conduite d'alimentation secondaire (27) est raccordée au récipient collecteur (8), via un filtre stérile (30).

4. Dispositif selon l'une quelconque des revendications précédentes, **caractérisé par** une conduite de rinçage (20) raccordée à la conduite de liaison (9), qui présente un troisième élément de raccordement (22) qui est relié à la conduite de rinçage (20) au moyen d'un filtre stérile (24).

5. Dispositif selon l'une quelconque des revendications précédentes, **caractérisé en ce qu'**un élément d'obturation (26) est disposé le long de la conduite de liaison (9) pour fermer la conduite de liaison (9).

6. Dispositif selon l'une quelconque des revendications précédentes, **caractérisé en ce que** sur la conduite d'alimentation (4), sur la conduite d'évacuation (13) et/ou sur la conduite de rinçage (20) est disposé respectivement un élément de commande (7, 15, 25, 42) pour commander le débit du fluide.

7. Dispositif selon l'une quelconque des revendications précédentes, **caractérisé en ce que** la conduite d'évacuation (13) est raccordée directement au récipient collecteur (8), en particulier au moyen d'un filtre à coagulation (16).

8. Dispositif selon l'une quelconque des revendications précédentes, **caractérisé en ce qu'**au moins deux récipients de prélèvement d'échantillons (17) sont raccordés au récipient collecteur (8).

9. Dispositif selon l'une quelconque des revendications précédentes, **caractérisé en ce que** le dispositif (3) est fermé de manière stérile.

10. Système de circulation de fluide comprenant un appareil de thérapie (2) et un dispositif (3) qui y est raccordé de manière stérile selon l'une quelconque des revendications précédentes.

11. Procédé pour former un système de circulation de fluide fermé, en particulier stérile, **caractérisé par** les étapes de procédé suivantes
- mise à disposition d'un dispositif selon l'une quelconque des revendications 1 à 9, couplage stérile du dispositif (3) avec un appareil de thérapie (2), en particulier un appareil d'aphérèse, le couplage stérile étant réalisé en raccordant de manière stérile le premier élément de raccordement (5) et le deuxième élément de raccordement (14) à l'appareil de thérapie (2) et en formant ainsi un système de circulation de fluide fermé et stérile.
